# EUROPEAN PATENT APPLICATION

(11) **EP 3 950 017 A1**
(43) Date of publication of application: **09.02.2022**
(21) Application number: 20778500.7
(22) Date of filing: 27.03.2020
(51) Int. Cl.: A61L 24/00, A61L 27/38, A61L 27/36, A61L 27/54

(54) **TISSUE FUSION COMPOSITION HAVING TISSUE ADHESION AND DIFFERENTIATION CHARACTERISTICS, AND PREPARATION METHOD THEREFOR**

(30) Priority: 28.03.2019 KR 20190035835; 25.03.2020 KR 20200036318
(71) Applicant: Ajou University Industry-Academic Cooperation Foundation, Suwon-si, Gyeonggi-do 16499 (KR)
(72) Inventor: MIN, Byoung-Hyun, Anyang-si Gyeonggi-do 14101 (KR)
(74) Representative: Roos, Peter
(86) International application number: PCT/KR2020/004253
(87) International publication number: WO 2020/197337

(57) **Abstract**

The present invention relates to a tissue fusion composition having tissue adhesion and differentiation characteristics, and a preparation method therefor. In the present invention, a tissue fusion composition in the form of a gel or sheet is prepared using stem cells and stem cell-derived extracellular matrix, and it has been confirmed that the composition adheres and binds superbly to biological tissues and can be differentiated into cartilage, bone, cornea, growth plate, and the like, and thus the composition can be used as an adhesive and a differentiation agent in regenerative therapy of damaged tissues or organs, therefore being ultimately and effectively usable as an agent for tissue fusion.

## Description

### TECHNICAL FIELD

The present invention relates to a tissue fusion composition having tissue adhesion and differentiation characteristics, and a preparation method therefor.

### BACKGROUND ART

Since the fibrin sealant was approved by the U.S. FDA in 1998, new tissue adhesives are constantly being developed every year. These tissue adhesives are spotlighted as a material that can replace techniques used in conventional surgical or medical operations such as suturing, clipping, and moxibustion.

Conventional surgical techniques, such as suturing, have strong tensile strength, but have disadvantages such as inducing pain in the patient and removal after the procedure. While the tissue adhesive has advantages such as quick adhesion, simple-to-use, and no need to be removed after the procedure, it has limitations in that it has low adhesiveness and elongation, and the adhesiveness is significantly lowered in the presence of moisture. Accordingly, research has been continued to overcome the limitations of the tissue adhesive as described above.

Medical tissue adhesives require biocompatibility because they come in direct contact with tissues, and because they are normally used in vivo, they must be non-toxic and non-hazardous to the human body and biodegradable materials under stricter conditions in case the adhesive flows into body fluids and blood.

Tissue adhesives that are currently commercialized and/or put into practical use are largely a cyanoacrylate adhesive, a fibrin adhesive, a gelatin adhesive, a polyurethane adhesive, and the like. The cyanoacrylate adhesive has recently been spotlighted in research on instant adhesives with high functionality and high performance. The cyanoacrylate-based tissue adhesive is a single material, cured by moisture at room temperature in a short time without an initiator, has a transparent appearance and a great adhesive strength, but is disadvantageous in that it is weak to shock and has a poor heat resistance. In addition, it is currently rarely used due to its severe toxicity, and is partially used in countries other than the United States, and its use is limited in some places due to tissue toxicity and fragility.

The fibrin adhesive has the advantage of being able to adhere quickly without being affected by moisture present at an adhesion site, there is no coagulation interference with platelets, and has excellent biocompatibility. However, it is disadvantageous in that its adhesive strength is weak, the biodegradation rate is fast, and there is a risk of blood infection.

Further, although the gelatin adhesive has a high tissue adhesion, formalin or glutaraldehyde used as a crosslinking agent has a disadvantage of causing tissue toxicity by crosslinking reaction with proteins in the living body. The polyurethane adhesive is advantageous in that it enhances adhesion to living tissue by absorbing water on a surface of the living tissue, is cured by reacting with water within minutes, and is biodegraded, but is disadvantageous in that aromatic diisocyanate, a synthetic raw material, is biotoxic.

On the other hand, adhesives developed so far have hardly contributed to fusion between tissues or between a tissue and implant. In other words, since the tissue is not permanently fused with the adhesive, the two tissues or the tissue and the implant fall off again after biodegradation of the adhesive. In order to overcome this shortcoming, cell therapeutic agents that supply therapeutic cells from the outside are being studied. In other words, by injecting cells between tissues or between a tissue and implant, the transplanted cells secrete extracellular matrix to attach the adhesion material. However, the cell therapies have no adhesive properties, so a separate adhesive or adhesion material is required while the cells are regenerating tissues.

As described above, the tissue adhesive requires adhesion between two tissues, and, ideally, it should serve as a cell therapeutic agent that can differentiate into cells of the target tissue to be attached and ultimately secrete extracellular matrix in order to permanently attach the two tissues. Such cell loaded tissue adhesive can be used for treatment of many tissue injuries in the body. For example, the injuries include muscle rupture, ligament damage, bone attachment, and cartilage regeneration. Therefore, development of a tissue adhesive having tissue adhesion and loaded with differentiated cells is expected to bring about a breakthrough in transplantation therapy.

### DESCRIPTION OF EMBODIMENTS

### TECHNICAL PROBLEM

It is an object of the present invention to provide a tissue adhesive product (TAP) composition having tissue adhesion and differentiation characteristics and a preparation method therefor.

### SOLUTION OF PROBLEM

In order to achieve the above object, the present invention provides a tissue fusion composition including stem cells and stem cell-derived extracellular matrix as an active ingredient.

In addition, the present invention provides a tissue for transplantation prepared by attaching two or more tissues with the composition.

In addition, the present invention provides a preparation method of a tissue fusion composition including the steps of (a) separating and culturing stem cells from fetal cartilage tissue; (b) obtaining a cell membrane including the cultured stem cells and their extracellular matrix; (c) centrifuging the obtained cell membrane to obtain a cell pellet; and (d) culturing the obtained cell pellet in a medium.

### ADVANTAGEOUS EFFECTS

In the present invention, a tissue fusion composition was prepared in the form of a gel or a sheet using stem cells and stem cell-derived extracellular matrix, and it has been determined that the composition has excellent adhesion and binding capability to biological tissues and has capability to differentiate into cartilage, bone, cornea, growth plates, etc. after transplantation. Thus, it can be used as an adhesive and an differentiation agent in regeneration therapy of damaged tissues or organs, and ultimately as a tissue fusion agent usefully.

### BRIEF DESCRIPTION OF DRAWINGS

FIG. 1 shows a result of determining adhesion strength of a cartilage tissue adhesive product (TAP) composition of the present invention.
FIG. 2 shows a result of determining whether or not, after transplanting a TAP labeled with the fluorescent expression factor PKH-26 into a cartilage damage model, the TAP is attached to a damaged site.
FIG. 3 shows results of safranin O staining and hematoxylin & eosin staining of TAPs prepared by culturing for 1 week, 2 weeks, and 3 weeks.
FIG. 4 shows results of confirming regeneration of cartilage damage through histological analysis after transplanting a TAP into a cartilage damage model.
FIG. 5 shows results of confirming differentiation into corneal cells using stem cells derived from fetal cartilage tissue.
FIG. 6 shows results of confirming differentiation into sheet-shaped corneal cells using a TAP.
FIG. 7 illustrates preparation of a growth plate injury model and a TAP transplantation method.
FIG. 8 shows results of confirming differentiation into cartilage tissues and growth plate tissues by a TAP in a growth plate injury model.
FIG. 9 shows results of confirming formation of collagen and glycoprotein by a TAP in a growth plate injury model.
FIG. 10 shows results of determining effects of improving length growth and angular deformity by a TAP in a growth plate injury model.
FIGS. 11 to 13 show results of confirming formation of cartilage tissues by a TAP in a growth plate injury model.
FIG. 14 shows results of confirming lumbar intervertebral disc regeneration by a TAP.
FIG. 15 shows a Gross image result of confirming cell differentiation after 7 days of culture of a fetal cartilage-derived stem cell sheet.
FIG. 16 shows Western blotting analysis results of determining expression levels of Myf5 and MyoD to confirm differentiation into myoblasts after 7 days of culture of fetal cartilage-derived stem cell sheets.
FIG. 17 shows results of confirming expression of Myf5 and MyoD by performing immunocytochemistry (ICC) to confirm differentiation into myoblasts after 7 days of culture of fetal cartilage-derived stem cell sheets.

### BEST MODE

The present invention provides a tissue fusion composition having tissue adhesion and differentiation characteristics including stem cells and stem cell-derived extracellular matrix as an active ingredient.

As used herein, the term "stem cell" may refer to a stem cell isolated from fetal cartilage tissue, and preferably a fetal cartilage derived progenitor cell (FCPC) isolated after complete digestion of the cartilage tissue using collagenase or the like.

As used herein, the term "stem cell-derived Extracellular Matrix" refers to a biopolymer aggregate consisting of molecules synthesized by cells from fetal cartilage-derived stem cells, secreted and accumulated outside the cells, including fibrous proteins such as collagen and elastin, complex proteins such as glycosaminoglycan, and cell adhesion proteins such as fibronectin and laminin.

As used herein, the term "cartilage" includes hyaline cartilage, fibrocartilage, or elastic cartilage, and is not particularly limited. It includes but not limited to articular cartilage, ear cartilage, nasal cartilage, elbow cartilage, meniscus, knee cartilage, costal cartilage, ankle cartilage, tracheal cartilage, occipital cartilage, and vertebral cartilage.

The composition may be differentiated into cornea, epithelium, growth plate, bone, cartilage, ligament, muscle, or skin tissues, but is not limited thereto.

The composition may be prepared in the form of a gel or a sheet, but is not limited thereto.

As used herein, the term "gel" is a jelly-like material and indicates a solid which has physical properties ranging from soft and weak to strong and rough and does not flow in a steady state. Most of the weight of the gel come from a liquid but it behaves as a solid due to its three-dimensional network structure.

The composition may be maintained at 25 to 40°C for 20 to 30 hours, but is not limited thereto.

In addition, the present invention provides a tissue for transplantation prepared by attaching two or more tissues with the composition.

The term "transplantation" as used herein generally refers to a process of transferring cells, tissues or organs of a donor to a damaged tissue or organ of a recipient, and in the present invention, it indicates application of the tissue fusion composition to a tissue defect, an injury, and a damaged site. Transplantation may be performed by methods known in the art. For example, it can be performed as a surgical operation, or a direct injection into an affected area.

In addition, the present invention provides a preparation method of a tissue fusion composition including the steps of: (a) separating and culturing stem cells from fetal cartilage tissue; (b) obtaining a cell membrane including the cultured stem cells and their extracellular matrix; (c) centrifuging the obtained cell membrane to obtain a cell pellet; and (d) culturing the obtained cell pellet in a medium.

The step (b) of obtaining a cell membrane may include obtaining the extracellular matrix together with the cells attached to the bottom without a cell separation step.

According to a culture period at the step (d), the compressive strength, adhesion, and applicability of the composition can be adjusted.

As used in the present invention, the term "applicability (spreadability)" refers to the property of spreading among physical properties, and refers to the property of spreading smoothly over the entire surface without forming a lump when applied to an affected area. In the present invention, the spreadability refers to a degree of spread per unit weight of a sample when a force of 5 N is applied to the sample vertically for 1 second at a speed of 1 mm/min.

The term "adhesion" as used in the present invention refers to the property of adhering a substance to the other substance, and in particular the property of attaching the substance to the affected area or the like without falling off when the substance is applied thereto. In the present invention, adhesion refers to the resistance until a material attached the affected area is separated from a jig with a diameter of 5 mm while pulling at a speed of 1.3 mm/min after contacting and attaching the material to the jig and the affected area.

The composition may be differentiated into cornea, epithelium, growth plate, bone, cartilage, ligament, muscle, or skin tissues, but is not limited thereto.

In addition, the present invention provides a tissue fusion composition having tissue adhesion and differentiation characteristics including stem cells and stem cell-derived extracellular matrix as an active ingredient.

As used herein, the term "regeneration" generally refers to an action to restore a part of an organism's body or recover its function to an original state when losing it by recreating a tissue or organ of the part. The simpler the system and the lower the degree of phylogenetic evolution, the stronger this regenerative ability.

### MODES FOR CARRYING OUT INVENTION

Hereinafter, the present invention will be described in detail with reference to examples. These examples are only for illustrating the present invention more specifically, and it will be apparent to those skilled in the art that the scope of the present invention is not limited by these examples.

### Example 1: Isolation and culture of stem cells derived from human fetal cartilage tissue

Cartilage-derived stem cells were isolated from the knee joint of a 12-15 week-old fetus (Source: IRB NO. AJIRB-CRO-07-139 approved by the Ethics Committee of Ajou University Hospital). Briefly, after washing cartilage tissue isolated from the knee joint with phosphate buffered saline (PBS) and adding a DMEM (Dulbecco's Modified Egle Medium, Gibco, Grand Island, NY) medium containing 0.2% (w/v) collagenase (Worthington Biochemical Corp., Lakewood, NJ), the cartilage tissue was cultured in a 37°C, 5% CO₂ incubator for 4 hours. Cartilage-derived stem cells released after complete digestion of the cartilage tissue were centrifuged at 1700 rpm for 10 minutes to obtain precipitated fetal cartilage-derived progenitor cells (FCPC), and then they were seeded in a tissue culture dish [150 mm (dia.) X 20 mm (h)] at a density of 1 X 10⁶ cells.

### Example 2: Preparation of tissue adhesive product (TAP) having tissue adhesion and differentiation characteristics

After diluting the cartilage stem cells obtained in Example 1 to a cell density of 2 X 10⁵ cells, they were cultured in a monolayer in the DMEM medium to which 10% fetal bovine serum (FBS), 50 units/mL penicillin, and 50 µg/mL streptomycin were added for 15 to 18 days. After culture, the medium was removed, and 0.05% trypsin-EDTA (Gibco) was added to obtain a cell membrane bound to extracellular matrix. To obtain the cell membrane to which the cell and the extracellular matrix were bound, the entire cell membrane including the cell and the extracellular matrix was obtained at once without separating the cells with a pipette after treatment with 0.05% trypsin-EDTA.

The cell membrane containing the obtained cells and extracellular matrix was put into a 50 ml tube containing a cartilage differentiation medium [1% antibiotic-antimycotic, 1.0 mg/mL insulin, 0.55 mg/mL human transferrin, 0.5 mg/mL sodium selenite, 50 µg/mL Ascorbic acid, 1.25 mg/mL bovine serum albumin (BSA), 100 nM dexamethasone, 40 µg/mL proline, and DMEM-HG added with 10 ng/ml TGF-β] and was centrifuged at 250 xg for 20 minutes to prepare a pellet-type construct.

The prepared cell pellet was placed in a culture dish, and a composition for tissue fusion was prepared by culturing for 1 week, 2 weeks, and 3 weeks in the 37°C, 5% CO₂ incubator, using a cartilage differentiation medium of the same composition as described above.

### Example 3: Measurement of adhesive strength of the tissue fusion composition

In order to determine adhesive strength of the TAP of Example 2, its adhesive ability was compared with that of the fibrin adhesive using a Universal Testing Machine (Model H5K-T, H.T.E, UK).

A patient's cartilage tissue, which had been discarded after joint arthroplasty, was donated together with a written consent. A cartilage damage model was prepared on the surface of the patient's cartilage tissue using a 6 mm biopsy punch, and the prepared TAP was inserted. Then, after attaching a jig with a diameter of 5 mm to the inserted TAP, the resistance was measured until the jig was separated from the TAP while pulling it at a speed of 1.3 mm/min.

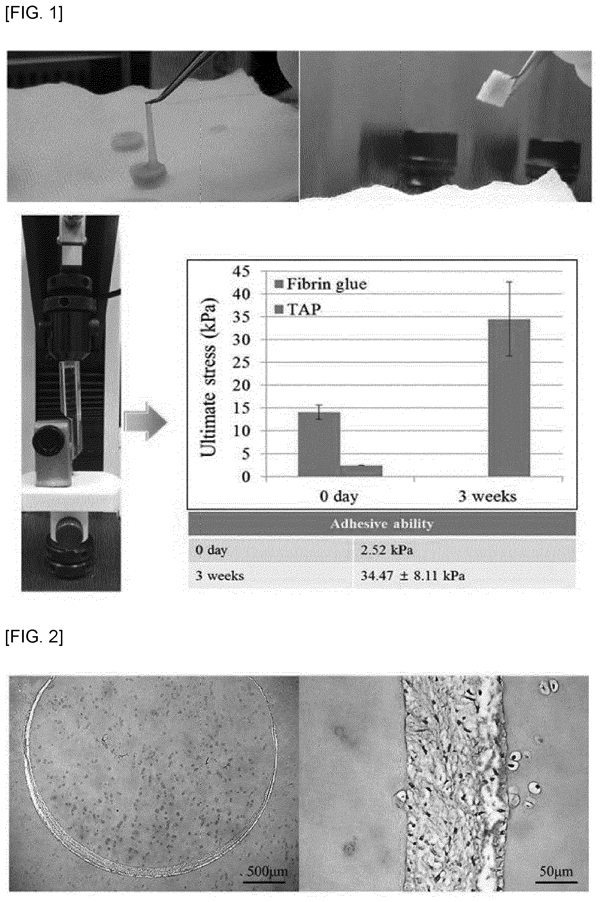

As a result, referring to FIG. 1, the adhesive strength of the TAP of the present invention was initially 2.52 kPa, which was weaker than that of the fibrin adhesive, but after 3 weeks, as differentiation into the tissue progressed, it was determined that the adhesive strength was 34.47 kPa, which was significantly superior to that of the fibrin adhesive.

### Example 4: Confirmation of in vivo adhesion of TAP labeled with the fluorescent factor PKH-26

PKH-26, a fluorescent expression factor labeled on the cell surface, was attached to the TAP to determine whether or not it was expressed in vitro or in vivo. The TAP labeled with the fluorescent expression factor PKH-26 was prepared and cultured for one week in vitro, and then it was determined that the fluorescence was well expressed, and then transplanted into a rabbit partial cartilage damage model. One week after transplantation, the knee at the transplant site was separated and sectioned to a thickness of 4 µm using a cryosectioning machine to prepare a slide.

As a result of observation using an optical microscope and a fluorescence microscope, as shown in FIG. 2, it was determined that the TAP remained at the partial cartilage damage site and the fluorescence expression factor was attached. From the above results, it was confirmed that the TAP of the present invention could be applied and attached to the affected area.

### Example 5: Confirmation of differentiation into cartilage tissue

Every one week during the TAP preparation process from the cell pellet of Example 2, it was fixed with 4% formalin, embedded in paraffin, and cut to a thickness of 4 µm, and then the cross section wad stained with Safranin O and hematoxylin & eosin (H&E) in order to detect accumulated sulfated proteoglycan.

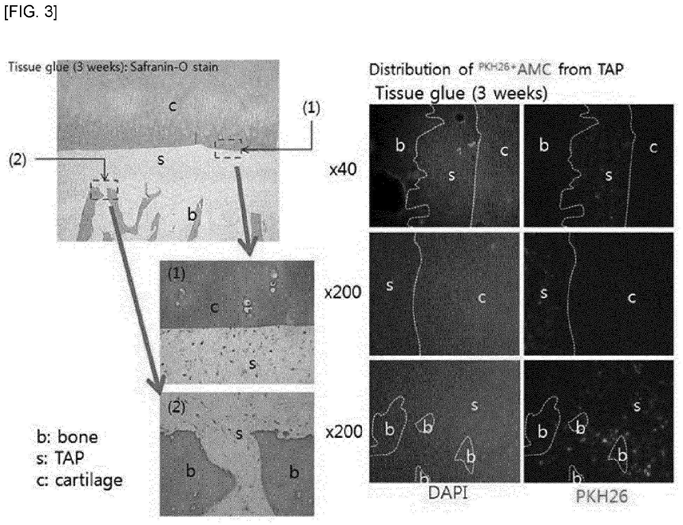

As a result, as shown in FIG. 3, it was determined that the cell spacing widened and the cell shape became similar to that of chondrocytes as time elapsed from 1 week to 3 weeks in the hematoxylin & eosin staining, and it was determined that the amount of proteoglycans increased and a lacuna, which can be seen in cartilage, was formed at 3 weeks in the safranin O staining.

In addition, after the TAP of the present invention was transplanted into a rabbit partial cartilage damage model, the knee at the transplantation site was separated and sectioned to a thickness of 4 µm using the cryosectioning machine to prepare a slide, followed by safranin O staining.

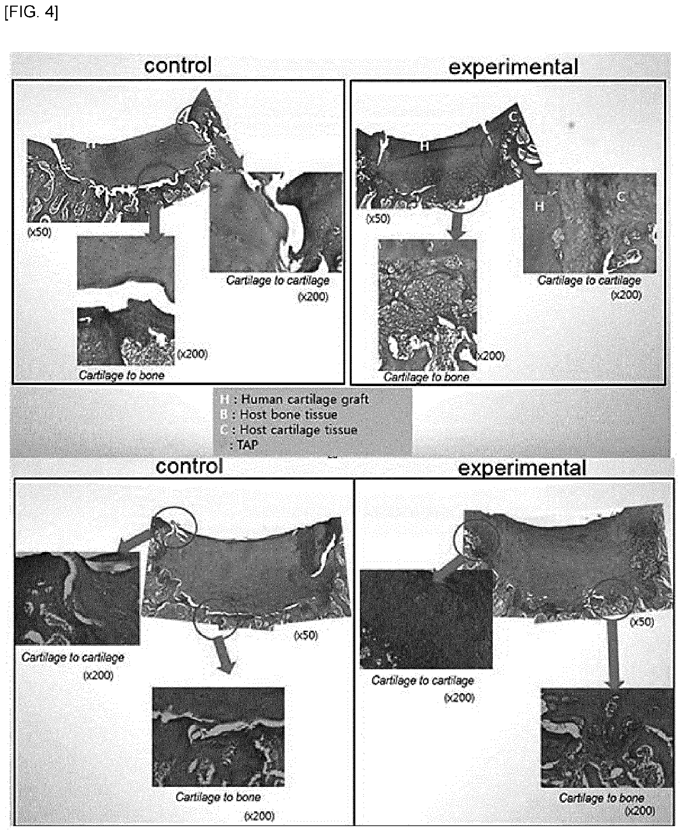

As a result, as shown in FIG. 4, when the boundary between the donor cartilage and the host cartilage, and the boundary between cartilage and bone were observed at 4 weeks after the transplantation, it was determined that integration between the cartilage and the cartilage and integration between the cartilage and the bone were excellent compared to those of a control group. After 8 weeks from the transplantation, it was determined that integrations between the cartilages and integration between the cartilage and the bone were almost complete and that the cartilage was restored similar to normal tissue to such an extent that the damaged area was hardly identified.

### Example 6: Confirmation of differentiation into corneal epithelial cells

### 6-1. Corneal epithelial cell differentiation

Fetal cartilage stem cells (3 X 10⁵ cells/cm²) were cultured in the 37°C, 5% CO₂ incubator using a low-glucose DMEM medium (HyClone, Logan, UT, USA) containing 2% KnockOut^{™} Serum Replacement (KnockOut^{™} SR, Thermo Fisher Scientific, Waltham, MA, USA), 10 ng/ml keratinocyte growth factor (KGF, Wako Pure Chemical, Japan), 10 ng/ml hepatocyte growth factor (HGF, Wako Pure Chemical, Japan), 20 ng/ml epidermal growth factor (EGF, Wako Pure Chemical, Japan), 0.5 µg/ml hydrocortisone (Sigma-Aldrich, St. Louis, USA), and 5 µM retinoic acid (Sigma-Aldrich, St.Louis, USA), and the medium was replaced every 2 days.

### 6-2. Immunocytochemistry

The cells were fixed with 4% paraformaldehyde for 20 minutes, and cultured with 0.1% Triton X-100 for 15 minutes and 5% BSA for 1 hour. After the cells were reacted with primary antibodies [anti-PAX6, anti-BCRP/ABCG2, anti-p63, anti-CK3/12 (1:200 dilution, Abcam, Cambridge, UK)] at room temperature for 2 hours, washed with PBS, and reacted with secondary antibodies [goat anti-mouse IgG H & L and goat anti-rabbit IgG H & L (1:1000 dilution, Alexa Fluor^{®} 488, Abcam, Cambridge, UK)] at room temperature for 1 hour. Thereafter, the cells were stained with 4,6-diamidino-2-phenylindole (DAPI) to visualize the nucleus, and observed using a fluorescence microscope (Mi8, Leica Microsystems, Wetzlar, Germany).

### 6-3. FACS

Cells (passage No. 5) were analyzed using corneal epithelial stem cell markers and corneal epithelial differentiation markers. The cells were reacted with primary antibodies [anti-CD34-FITC (BD Biosciences, San Jose, CA, USA), anti-CD105 (BD Biosciences, San Jose, CA, USA), anti-PAX6, anti-BCRP/ABCG2, anti-p63, anti-CK3/12 (1:500 dilution, Abcam, Cambridge, UK)] at room temperature for 1 hour, washed with PBS, and then reacted with secondary antibodies [goat anti-mouse IgG H & L and goat anti-rabbit IgG H & L (1:1000 dilution, Alexa Fluor 488, Abcam, Cambridge, UK)] at room temperature for 1 hour. Stained cells were analyzed by flow cytometry (Becton Dickinson FACSavantage).

### 6-4. Western blot

After collecting cells, the cells were lysed by reaction with a RIPA buffer added with a protease inhibitor (Rockland Immunochemicals, Pennsylvania, USA) at 4°C for 30 minutes. The cell lysate was centrifuged at 12,000 g at 4°C for 15 minutes, and then the protein was quantified. SDS-PAGE was performed using 20 µg of protein and it was transferred to a PVDF membrane. Then, the PVDF membrane was reacted with primary antibodies [actin (1:1000) (GeneTex Inc., California, USA), anti-PAX6, anti-BCRP/ABCG2, anti-p63, and antiCK3 (1:200, Abcam, Cambridge, UK)] at room temperature for 2 hours, and blocked with skim milk to prevent non-specific binding. Then, after reaction with secondary antibodies [HRP-conjugated goat anti-rabbit IgG and goat anti-mouse IgG (1:1000, GeneTex Inc., California, USA)] for 1 hour, bands were visualized using a chemiluminescence kit (ECL kit, Bio-Rad, Hercules, CA, USA) and an image analysis was performed using a Chemiluminescence system (Fusion SL2, VILBER LOURMAT, France) and Image J (NIH, USA).

### 6-5. Histology and Immunohistochemistry

After fixing a sample with 4% formaldehyde (Duksan Chemical, Korea), it was embedded in paraffin wax (Merck, Darmstadt, Germany). After making a slide section with a thickness of 4 mm, it was stained with hematoxylin & eosin to observe a fetal cartilage stem cell sheet and cell morphology in vivo.

For immunohistochemical analysis of CK3 and human nucleus, it was reacted with methanol added with 3% hydrogen peroxide (Duksan Chemical, Korea) for 10 minutes, and then reacted with a pepsin solution (Golden Bridge International, Inc., Mukilteo, WA, USA) for 10 minutes. After blocking with PBS added with 1% BSA, it was reacted with primary antibodies [anti-CK3 (1:100, Abcam, Cambridge, UK), anti-human nucleus (1:100, Millipore, Massachusetts, USA)] at room temperature for 1 hour and 30 minutes, reacted with a secondary antibody (biotinylated-anti mouse IgG (SPlink HRP Detection Kit; Golden Bridge International, Inc., Mukilteo, WA, USA)) for 30 minutes, and then reacted with a HRP-conjugated streptavidin solution for 30 minutes. Finally, before mounting, it was reacted with a 3,3'-diaminobenzidine (DAB) solution (Golden Bridge International, Inc., Mukilteo, WA, USA) and then counterstained with a Mayer's hematoxylin (YD Diagnostics, Seoul, Korea).

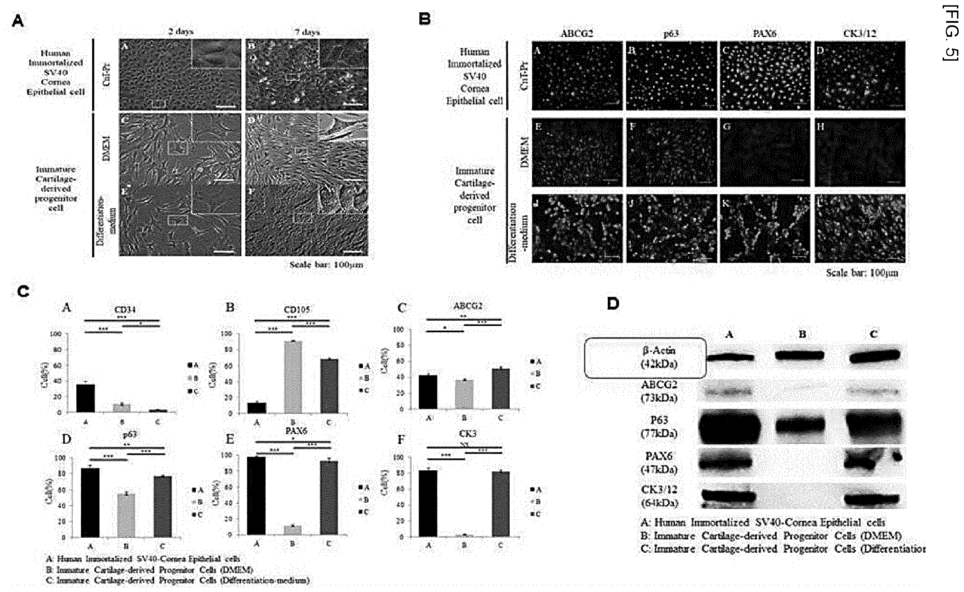

As a result, as shown in FIG. 5A, the morphology of the fetal cartilage stem cells cultured in the differentiation medium was compared with that of SV40 corneal epithelial cells. The SV40 corneal epithelial cells were cultured in Cnt-Pr medium. As a result, no morphological difference was observed between the two cells, and it was determined that the morphology of the fetal cartilage stem cells was similar to that of the corneal epithelial cells.

As shown in FIG. 5B, as a result of comparing the fetal cartilage stem cells cultured in the differentiation medium using the corneal epithelial stem cell markers (ABCG2, p63) and the corneal epithelial differentiation markers (PAX6, CK3/12) with the SV40 corneal epithelial cells, it was determined that ABCG2, p63, PAX6, and CK3/12 were expressed in the SV40 corneal epithelial cells. Further, it was determined that PAX6 and CK3/12 were not expressed and ABCG2 and p63 were expressed low in fetal cartilage stem cells cultured in a normal medium while ABCG2, p63, PAX6, and CK3/12 were all highly expressed in the fetal cartilage stem cells cultured in the differentiation medium. Consequently, it was determined that expression of the corneal epithelial stem cell markers and the corneal epithelial differentiation markers was induced in the fetal cartilage stem cells.

As shown in FIGS. 5C and 5D, as a result of comparing the fetal cartilage stem cells cultured in the differentiation medium using the stem cell markers (CD34, CD105), the corneal epithelial stem cell markers (ABCG2, p63), and the corneal epithelial differentiation markers (PAX6, CK3/12) with the SV40 corneal epithelial cells, it was determined that expression of the corneal epithelial stem cell markers and the corneal epithelial differentiation markers was induced in the fetal cartilage stem cells cultured in the differentiation medium, similarly to the SV40 corneal epithelial cells.

### Example 7: Corneal Epithelial Animal Model with Chemical Burns

### 7-1. Preparation of fetal cartilage stem cell sheet

Fetal cartilage stem cells were passaged through trypsinization (passages 4 to 5). Non-adherent cells were washed by changing the medium 2-3 times with fresh ones. Thereafter, the fetal cartilage stem cells (2 X 10⁵ cells/cm²) were cultured in a sheet-medium with a high glucose DMEM medium including 100 U/ml penicillin G and 100 µg/ml streptomycin (HyClone), insulin-transferrin-selenium (ITS, Gibco BRL, NY, USA), 50 µg/ml ascorbate-2 phosphate, 100 nM dexamethasone, 40 µg/ml proline, 1.25 mg/ml bovine serum albumin, and 100 µg/ml sodium pyruvate (Sigma-Aldrich, St. Louis, USA), and cultured in the 37°C, 5% CO₂ incubator for 3-4 days until the fetal cartilage stem cells fell off the culture dish.

### 7-2. Corneal epithelial animal model with chemical burns

Animal experiments were carried out after obtaining an approval from the Ajou University Animal Experiment Ethics Committee. Corneal limbal stem cell deficiency (LSCD) was induced in the right eye of each rabbit (18 rats). After anesthetizing the rabbit by intramuscular injection of a mixture of ketamine and zoletyl, a filter paper with a diameter of 8 mm was saturated with 1 M NaOH, placed on the corneal limbus for 30 seconds, and washed with saline for 1 minute, and this process was repeated three times. The fetal cartilage stem cell sheet and PKH26-labeled fetal cartilage stem cell sheet were placed on the cornea with forceps and sutured with 6-0 black silk sutures at the top, bottom, left and right sides.

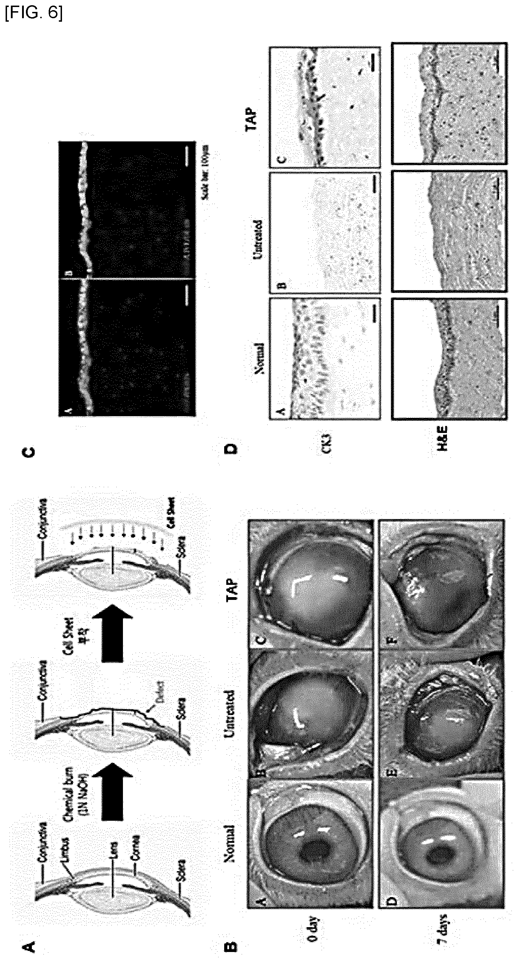

As shown in FIG. 6B, as a result of analyzing effects of the fetal cartilage stem cell sheets in the rabbit animal model with chemical burns in the right eye, the normal corneal epithelium was observed on days 0 and 7 in the normal group (A, D). Damaged corneal epithelium was observed on days 0 and 7 in a control group (B, E), while the corneal epithelium was observed on days 0 and 7 in the case of being treated with the fetal cartilage stem cell sheet (C, F). Thus, it was determined that the damaged corneal epithelium was treated by the fetal cartilage stem cell sheet.

In addition, as shown in FIG. 6C, as a result of analyzing the sheet position and viability of the fetal cartilage stem cells on the 7th day after transplantation, the PKH26-labeled fetal cartilage stem cell sheet was observed at the damaged site (A), and it was determined that the fetal cartilage stem cell sheet was alive at the damaged site (B).

As shown in FIG. 6D, as a result of analyzing a healing ability using CK3, the corneal epithelial differentiation marker, on the 7th day after transplantation, it was determined that the fetal cartilage stem cell sheet was located at the damaged site and exhibited a healing effect on corneal damage.

### Example 8: Confirmation of differentiation into growth plate tissue

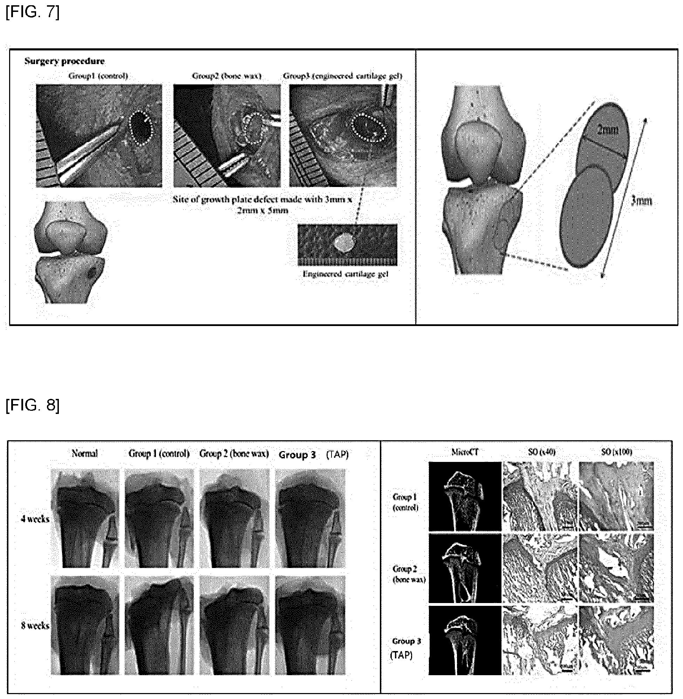

As shown in FIG. 7, the rabbit growth plate was pierced obliquely twice with a 2 mm punch, and the TAP of the present invention was transplanted. After that, the transplanted site was separated and sectioned to a thickness of 4 µm using the cryosectioning machine to prepare a slide. Only the growth plate was damaged in a negative control group. As a positive control group, bone wax, which is currently most used in clinical practice, was used.

Consequently, as shown in FIG. 8, it was determined that cartilage was formed in the group transplanted with the TAP of the present invention after 8 weeks. The bone wax used as the positive control group has an effect of filling the transplantation space, but it has a limitation in that the transplanted material is maintained as bone tissues and results in angular deformity of the bone. On the contrary, in the case of the TAP of the present invention, it was determined that normal cartilage was regenerated to such an extent that the transplantation site was not distinguishable to the naked eye.

In addition, by safranin O and hematoxylin & eosin staining, it was determined that bone tissues were formed at the damaged transplantation sites in the negative and positive control groups, while cartilage tissues were obviously located at the growth plate position in the group transplanted with the TAP of the present invention.

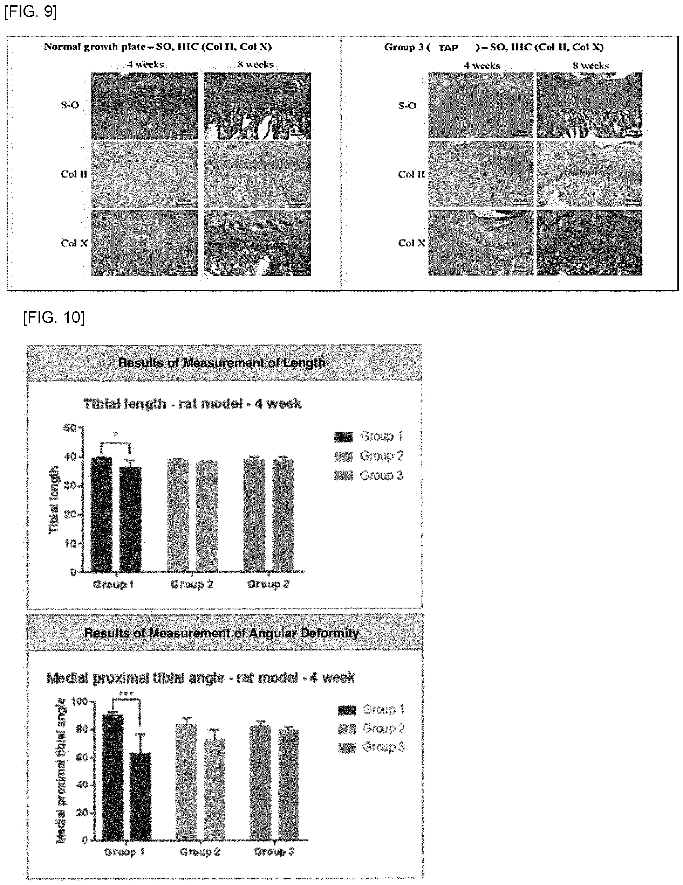

In addition, as shown in FIG. 9, as a result of immunostaining, it was determined that collagen and glycoproteins were formed in the TAP-transplanted group similarly to the normal group, and in the case of collagen X, which is the stage of bone formation, it was formed after 8 weeks. Thus, it can be expected that ossification would occur late or be prevented.

### Example 9: Confirmation of cartilage formation ability in growth plate

The TAP of the present invention was transplanted in a rabbit growth plate injury model, and ossification capacity was determined in cartilage of the growth plate (Group 1: negative control group, Group 2: positive control group (bone wax), Group 3: TAP-treated group) (left bar: average length, right bar: result of Example).

As a result, as shown in FIG. 10, a difference in length growth was observed only in the negative control group, and the most angular deformity was observed in the negative control group. In the positive control group in which the bone wax was transplanted, the angular deformity was also observed.

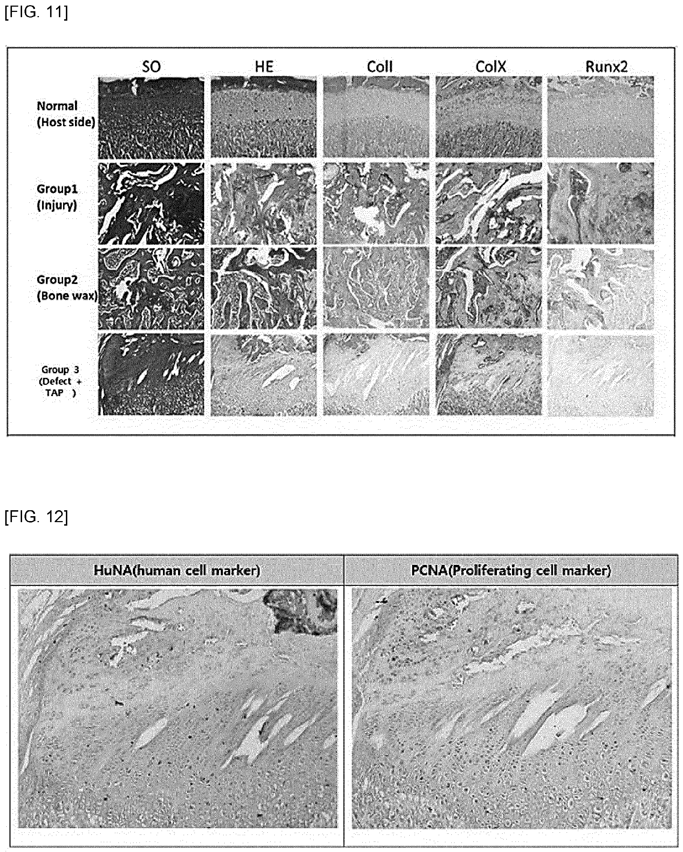

In addition, as shown in FIG. 11, it was determined through safranin O and hematoxylin & eosin staining that bone tissues were formed at the injured sites in the negative and positive control groups, but the TAP of the present invention was maintained as cartilage tissues.

Overall, in the 4-week results, it was determined that the injured growth plate site was filled with cartilage tissues only in the TAP-transplanted group, unlike the negative control group and the positive control group, while a possibility of length growth was confirmed by expression of a hypertrophic marker in a growth site.

Further, as shown in FIG. 12, transplanted tissues were identified by staining the group transplanted with the TAP with HuNA, and proliferating cells were identified by staining with PCNA.

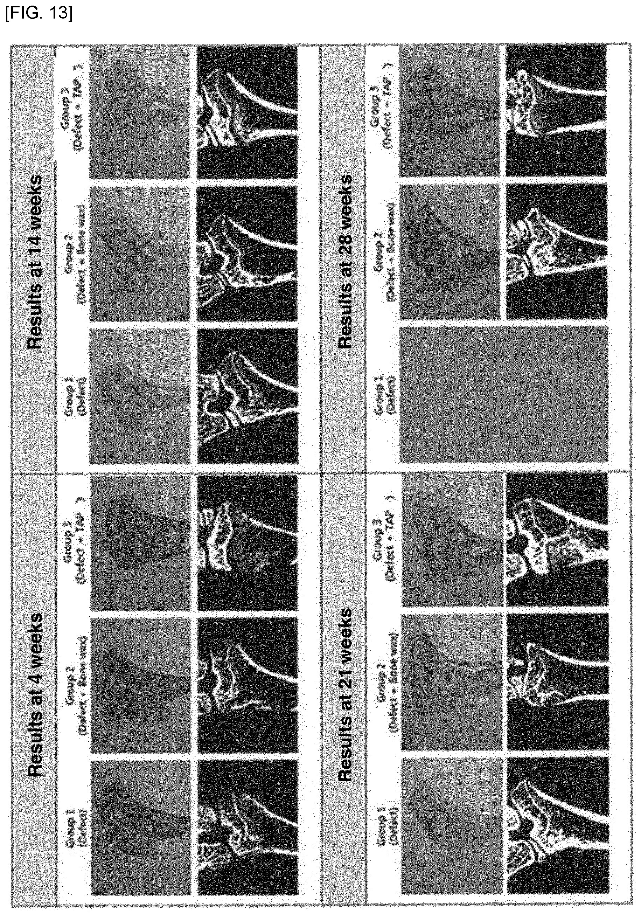

Furthermore, as shown in FIG. 13, as a result of performing safranin O and uCT at 4, 14, 21, and 28 weeks after transplantation, almost the same trend was observed in the group transplanted with the TAP from results at 4, 14, 21, and 28 weeks. In the negative control group and the positive control group, it was determined that no cartilage tissues were found at the growth plate injury sites, and that bone tissues were formed in the u-CT results. In particular, in the case of the negative control group, it was determined that the angular deformity occurred at all weeks.

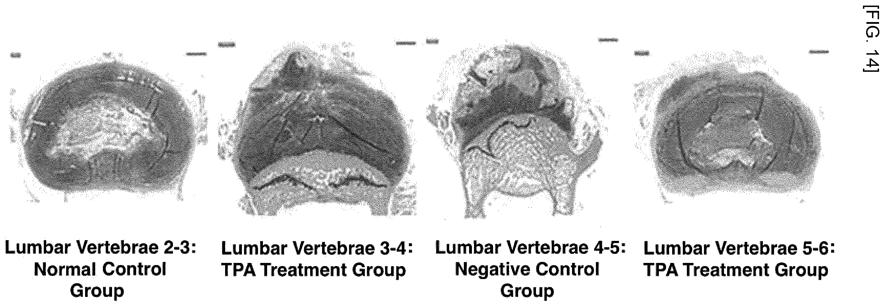

In addition, as shown in FIG. 14, as a result of comparing regeneration of lumbar intervertebral discs through histological examination (lumbar vertebrae 2-3: a normal control group, lumbar vertebrae 3-4: a TAP treatment group, lumbar vertebrae 4-5: a negative control group, lumbar vertebrae 5-6: a TAP treatment group), it was determined that defects of fibrous annulus of the intervertebral discs between the lumbar vertebrae 3-4 and the lumbar vertebrae 5-6 were restored.

### Example 10: Confirmation of muscle differentiation effect using fetal cartilage-derived stem cells sheet (TAP-C)

In order to make artificial muscle tissue that can be used for muscle regeneration using a fetal cartilage-derived stem cells sheet in the laboratory, TAP-C was used to determine a muscle differentiation effect.

Fetal cartilage-derived stem cells (FCSC) were dispensed in a 6-well plate, and sheet groups consisted of a 3 × 10⁵/well group and a 2 × 10⁶/well group to determine differences in differentiation according to the density and thickness of the cell sheet. As a medium, a High-glucose DMEM (Hyclone) added with 10% FBS was used.

After 1 day of culture, the medium was replaced with a Myoblast-differentiation induction medium. The medium was prepared by adding 1 ng/ml transforming growth factor-β1 (TGF-β1; R&D systems), non-essential amino acids (NEAA; Invitrogen), and insulin-transferrin-selenium (ITS; Gibco) to DMEM/nutrient mixture F-12 (Invitrogen). Culture was performed for 7 days to differentiate FCSCs into myoblasts.

After 7 days of culture, to confirm differentiation into myoblasts, western blotting and immunocytochemistry (ICC) were performed to determine expression levels of Myf5 and MyoD.

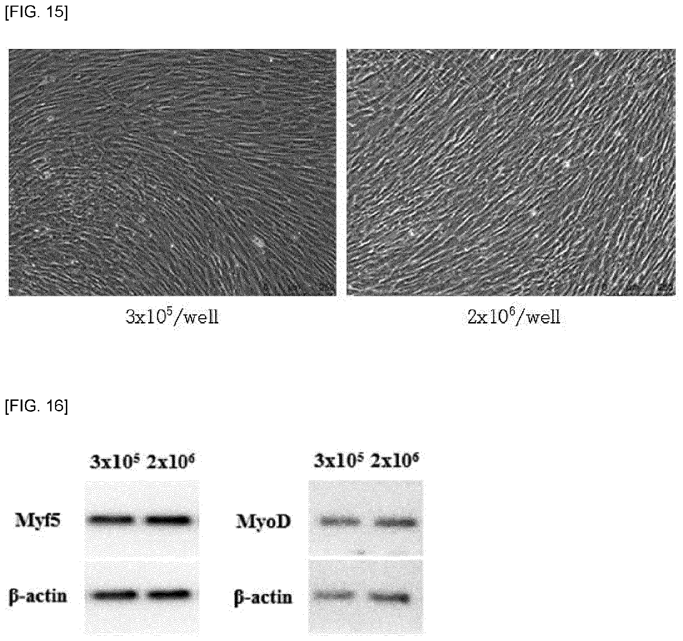

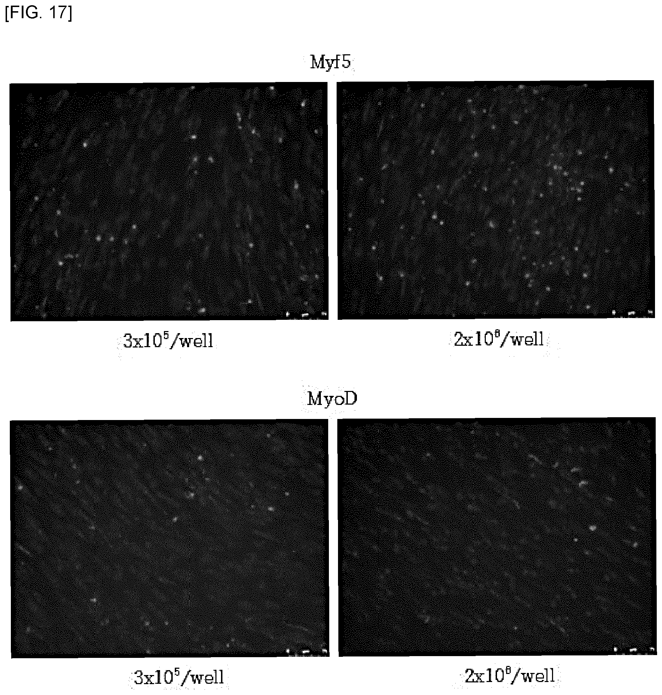

As a result, as shown in FIGS. 15 to 17, the expression of Myf5 and MyoD, which act as major factors in the early stage of muscle differentiation, appeared, and it was determined that the FCSC sheet was differentiated into myoblasts.

Although the present invention has been described in detail above with reference to particular embodiments and examples, it will be apparent to those skilled in the art that these specific descriptions are merely preferred embodiments and the scope of the present invention is not limited thereto. Accordingly, the substantial scope of the present invention will be defined by the appended claims and their equivalents.

The scope of the present invention is indicated by the following claims, and all changes or modifications derived from the spirit and scope of the claims and their equivalents should be construed as being included in the scope of the present invention.

## Claims

1. A tissue fusion composition having tissue adhesion and differentiation characteristics, comprising stem cells and stem cell-derived extracellular matrix as an active ingredient.

2. The tissue fusion composition according to claim 1, wherein the tissue is selected from the group consisting of cornea, epithelium, growth plate, bone, cartilage, ligament, muscle, and skin tissues.

3. The tissue fusion composition according to claim 1, wherein the composition is in the form of a gel or a sheet.

4. A tissue for transplantation prepared by attaching two or more tissues by the composition according to claim 1.

5. A preparation method of the tissue fusion composition according to claim 1 comprising:
(a) separating and culturing stem cells from fetal cartilage tissue;
(b) obtaining a cell membrane comprising the cultured stem cells and their extracellular matrix;
(c) centrifuging the obtained cell membrane to obtain a cell pellet; and
(d) culturing the obtained cell pellet in a medium.

6. The method according to claim 5, wherein the step (b) of obtaining a cell membrane comprises obtaining the extracellular matrix together with the cells attached to the bottom without a cell separation step.

7. The method according to claim 5, wherein the compressive strength, adhesion, and applicability of the composition are adjusted according to a culture period at the step (d).

8. The method according to claim 5, wherein the tissue is selected from the group consisting of cornea, epithelium, growth plate, bone, cartilage, ligament, muscle, and skin tissues.

9. A tissue fusion composition having a tissue adhesion characteristic comprising stem cells and stem cell-derived extracellular matrix as an active ingredient.
